# EUROPEAN PATENT APPLICATION

(11) **EP 2 727 609 A1**
(43) Date of publication of application: **07.05.2014**
(21) Application number: 12191366.9
(22) Date of filing: 06.11.2012
(51) Int. Cl.: A61L 9/03

(54) **Air treatment device for the vaporization of an air freshening substance**

(71) Applicant: The Procter & Gamble Company, Cincinnati, OH 45202 (US)
(72) Inventor: Ques Ramos, Victor, 1950 Kraainem (BE); Bartolucci, Stefano, London, SW15 3LX (GB)
(74) Representative: Pierce, Christopher James

(57) **Abstract**

An air treatment device (1) for the vaporization of an air freshening substance, the device comprising: a replaceable cartridge (2) comprising a reservoir (3), for containing the air freshening substance, and a wick (4), in fluid communication with the reservoir, having a top end (4a) and a bottom end (4b) defining a wick height; a heating means (5) for heating the wick (4) to a predetermined temperature to vaporize the air freshening substance; a heating means support (6) for retaining the heating means (5); and a housing (7) enclosing at least the heating means (5) and heating means support (6) therein, and having at least one opening for allowing the vaporized air freshening substance to exit the air treatment device, whereby said heating means support (6) is arranged to displace in a direction perpendicular to said wick (4), upon actuation of an actuating means (9), such that the evaporation rate is varied.

## Description

### FIELD OF INVENTION

The present invention relates to air treatment devices, generally of the electrical evaporator type, for the vaporization of air freshening substances and the like, typically operable via connection to a power source such as an electrical wall outlet, car power jack, battery and the like. The air freshening substances typically comprise one or more volatile components capable of masking and/or reacting with malodors present in the air, typically to eliminate or reduce the perceptibility of such malodors from the air.

### BACKGROUND OF THE INVENTION

Air treatment devices utilizing heat as a means to vaporize an air freshening substance are well known in the industry of air care. A variety of devices comprising heaters located proximal to a wicking element carrying a liquid substance exist, some of which further include a means of varying the rate of evaporation of the substance by enabling movement of the heater up and down the height of the wick. An example being, WO 01/39809 A1.

Other devices exist wherein the rate of evaporation of a substance is varied by displacement of the wick towards and away from the wick by bending the wick in a lateral direction with respect to the heater. An example being WO 2005/079874 A1.

However, the above mentioned devices incur problems if the desired operating temperature is increased.

Indeed, it is desirable to increase the operating temperature of such devices to temperatures typically above 90°C in order to enable a more extensive use of certain components, in an air freshening substance, having lower volatility (so called "base note" technology) to consequently provide better noticeable character over time. Such requirement becomes ever so more important for single chamber devices emitting a single air freshening substance or lacking complex and costly heat regulation processes that provide alternating emission at predetermined cycles aimed at enhancing fragrance character and limiting wick clogging.

Solely increasing the operating temperature in the abovementioned devices causes problems of for example melting of the housing, particularly in voltage fluctuations caused by failure within 120V residential electrical systems where loss of a single connection can allow a 120V appliance to see the full 240V source coming into the residence, as well as fatigue and/or swelling of the wick, both of which may not only limit the effectiveness of the device but may also result in catastrophic failure hazardous to the user and neighboring objects.

Therefore, there still remains a need for an air treatment device that simply, cheaply and effectively enhances the perceptible character of a vaporized air freshening substance and is operable at higher temperatures whilst overcoming the problems associated with known devices.

The present invention provides an air treatment device, for the vaporization of an air freshening substance, that overcomes the above problems.

An advantage of the present invention is that by particularly positioning a heat-resistant heating means support and arranging it such that it displaces in a direction perpendicular to the wick height, the wick is relieved from stresses that may otherwise be further increased by several magnitudes with the addition of more heat whilst at the same time ensuring appropriate heat insulation.

Other advantages of the present invention will become apparent with reference to the drawings and detailed description herein.

### SUMMARY OF THE INVENTION

The present invention relates to an air treatment device () for the vaporization of an air freshening substance, said device comprising: a replaceable cartridge comprising a reservoir, for containing said air freshening substance, and a wick, in fluid communication with said reservoir, having a top end and a bottom end defining a wick height; a heating means for heating said wick to a predetermined temperature to vaporize the air freshening substance drawn towards said top end by said wick; a heating means support for retaining said heating means; and a housing enclosing at least said heating means and heating means support therein, and having at least one opening for allowing the vaporized air freshening substance to exit the air treatment device, wherein said housing is slidably connectable to said heating means support but not connectable to said heating means, wherein at least a portion of the heating means support is interposed between the housing and the heating means and wherein the heating means is substantially interposed between said wick and said heating means support, the heating means support being arranged to displace in a direction perpendicular to said wick height, upon actuation of an actuating means connectable to said heating means support, such that the evaporative rate of the air freshening substance is varied, and wherein said heating means support is heat-resistant.

In a second aspect, the present invention relates to method of vaporizing an air freshening substance.

### BRIEF DESCRIPTION OF THE FIGURES

Fig. 1 is an exploded view of an embodiment of the present invention.
Fig. 2 is a front view of an embodiment of the present invention.
Fig. 3 is a cross section of Fig. 2 about line A-A.
Fig. 4 is a cross section of Fig. 2 about line A-A, illustrating the movement of the heating means support, according to an embodiment of the present invention.
Fig. 5 is a schematic illustrating the over-voltage scenario.

### DETAILED DESCRIPTION OF THE INVENTION

As used herein "air freshening substance" means any suitable composition that reduces odors in air, and/or reduces the impression of odors in the air by masking, layering or including malodor counteractant perfume raw materials inta the composition.

As used herein "perception" means a response to a sensory stimuli, such as the sense of smell, that triggers an emotional response, such as the identification of an odor.

As used herein "heat-resistant" means that the item referred to comprises a material having a structural relative temperature index (str. RTI) of greater than 150°C, preferably greater than 200°C based on 1.5 mm minimum thickness, as measured using the test method herein.

As used herein "habituation" means a state in the olfactory perception of a material, for example a fragrance component in an air freshening substance, by a receptor, typically a human, who becomes accustomed to the olfactory characteristics of the material following prolonged exposure of said same material such to limit or even negate the perceivable olfactory presence of said material.

As used herein "detection level of a human" means the level at which an average human is able to perceive the presence of a material, for example in an air freshening substance, to a sufficient extent to stimulate an emotional response resulting in the human experiencing a sensation such as registering of a smell and/or odor.

As used herein "wick," means an element used to transport the liquid air freshening substance to be dispensed. Such element typically will include some material, as well as the space created by pores contained therein. As used herein, the term "pores" refers to the cavities formed within the wick material itself. As will be discussed in greater detail below, "pore size or average pore size" is used to describe the average diameter of a sample of pores of the wick material, and is expressed in microns. Also described in greater detail below, "porosity or pore volume" refers to the ratio of the volume of all the pores of the wick material to the overall volume of the wick itself (pores and wick material), and is generally expressed herein as a percentage.

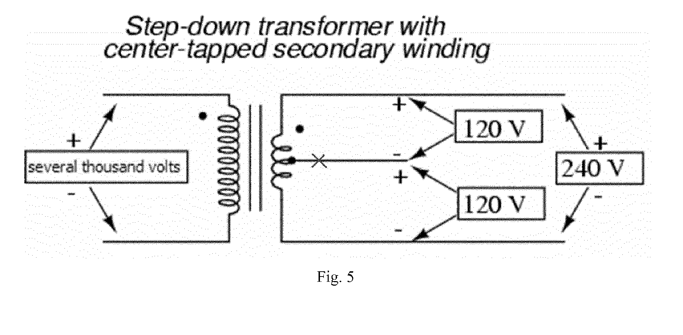

As used herein "overvoltage" means a condition in which the voltage in the circuit in which the device operates is raised above its upper specification limit. The condition of overvoltage can be particularly severe for 120V networks where a house wiring fault could allow a 120V appliance to see the full 240V source. With reference to Fig 5, 120V residential electrical systems bring a 240V to each house. The transformer would supply one to five houses. The center of this source is tapped to allow supply of 120 V appliances. If this center tap is lost, then the 240V no longer divides evenly and the voltage at each power socket in the residence will be outputting anything from 0 to 264 V (the actual value of overvoltage applied is calculated as the full available voltage of the entire transformer secondary multiplied by 110% - the 10% increase over the normal rating is applied based upon the tolerance of the power system and is generally applied by Underwriters Laboratories (UL) and International Electrotechnical Commission (IEC) during electrical safety testing). The actual value actually depends on the various loads (lights, TVs, cooking appliances) connected to each branch.

Various embodiments will now be described to provide an overall understanding of the principles of the structure, function, manufacture, and use of the air treatment device and methods disclosed herein. One or more examples of these embodiments are illustrated in the accompanying drawings. Those of ordinary skill in the art will understand that features described or illustrated in connection with one example embodiment can be combined with the features of other example embodiments without generalization from the present disclosure.

### AIR TREATMENT DEVICE

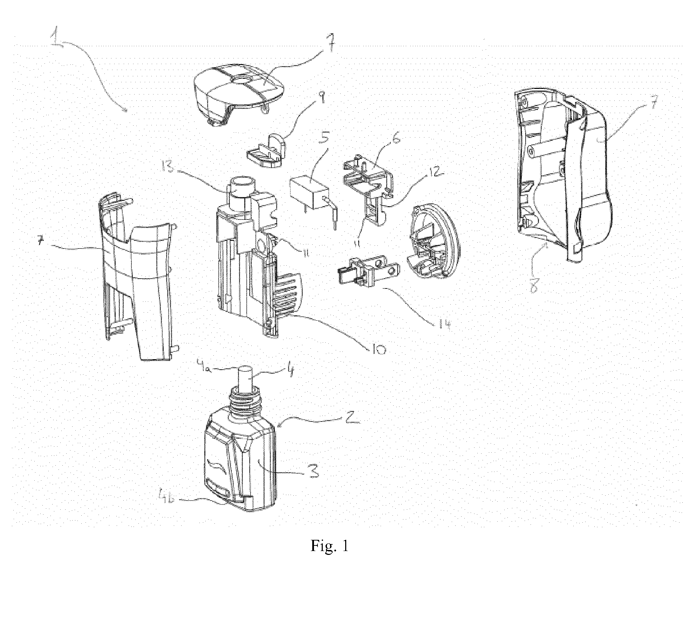

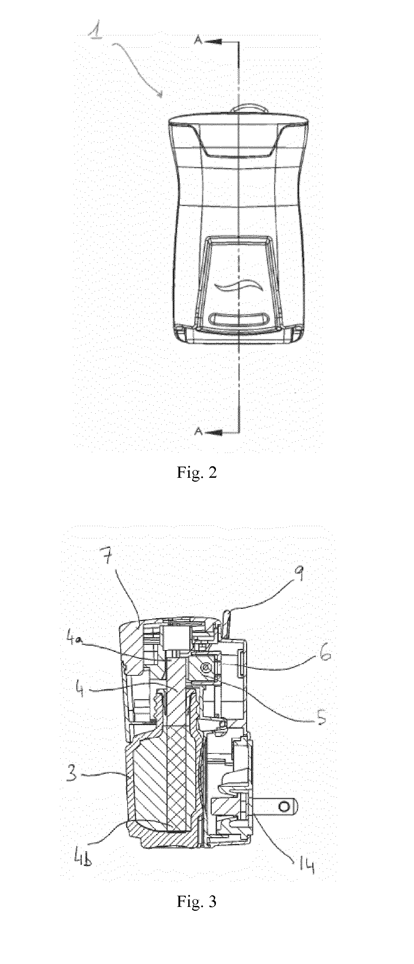

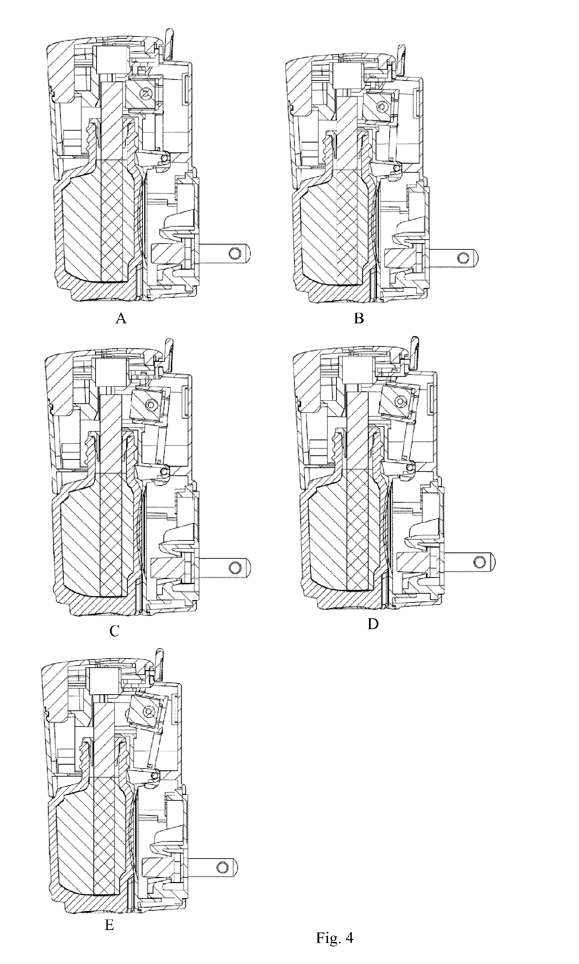

As shown in Fig.1 to Fig.4, the invention is directed to an air treatment device **1** for the vaporization of an air freshening substance, said device comprising: a replaceable cartridge **2** comprising a reservoir **3,** for containing said air freshening substance, and a wick **4,** in fluid communication with said reservoir **3,** having a top end **4a** and a bottom end **4b** defining a wick height; a heating means **5** for heating said wick **4** to a predetermined temperature to vaporize the air freshening substance; a heating means support **6** for retaining said heating means **5;** and a housing **7** enclosing at least said heating means **5** and heating means support **6** therein. The housing **7** having at least one opening for allowing the vaporized air freshening substance to exit the air treatment device **1.** The housing **7** may form a semi-enclosed recess **8** into which the replaceable cartridge **2** is insertable, preferably such that at least a portion of the replaceable cartridge **2** remains exposed to ambient air once fully inserted into the housing **7.**

The housing **7** may be moveably connectable, preferably directly connectable, to said heating means support **6** but not to said heating means **5.** Such ensures that the housing **7** is not directly exposed to the heat generated by the heating means **5** whilst minimizing the number of components and thus simplifying manufacture and reducing cost.

At least a portion, preferably a substantial portion, of the heating means support **6,** is interposed between the housing **7** and the heating means **5** and the heating means **5** is substantially interposed, preferably interposed, more preferably entirely interposed, between said wick **4** and said heating means support **6.** By "substantial portion" or "substantially" it is herein meant that at least 65%, preferably at least 70%, more preferably at least 80%, even more preferably at least 90%, most preferably at least 95%, of the total surface area, of the feature referred to, is positioned in the manner described (e.g. at least 90% of the total surface area of the heating means support is interposed between the housing and the heating means, etc.). Such ensures further limiting the exposure of the housing to heat in a simple and cost effective structure.

The heating means support 6 is arranged to displace in a direction substantially perpendicular to said wick height, upon actuation of an actuating means 9 connectable, preferably connected, to said heating means support **6,** such that the evaporative rate of the air freshening substance is varied. The wick height typically extending parallel to the axis of the wick **4** that crosses both top end **4a** and bottom end **4b** of the wick **4.** Such ensures, not only effectively controlling the heat through the housing, but also reliably varying the intensity of the air freshening substance being emitted, by varying the evaporation rate, whilst having minimal impact on the wick. A further advantage is that greater flexibility is achieved, allowing broader ranges of displacement which in turn permit more accurate intensity control and higher operating temperatures.

Preferably, the heating means support **6** is moveably coupled to the housing **7** and/or a cartridge support **10,** preferably coupled to the cartridge support **10,** at a pivot point **11** via an arm **12** which length may be selected to provide the desired displacement of the heating means support **6** relative to the wick **4** typically upon movement of an actuating means **9** fixedly connected to the heating means support **6** and moveably and/or slidably connected to the housing **7.** The length of the arm **12** may be from 10mm to 30mm, preferably from 15mm to 25mm, even more preferably from 20mm to 25mm, most preferably from 21mm to 23mm, measured from the pivot point **11** to the half way point of the height of the heating means **5.** The displacement of the heating means support **6** may therefore be a rotational displacement about the pivot point **11.** An advantage of this embodiment is simplicity of manufacture whilst ensuring accurate intensity control and structural stability and integrity of the components of the device.

The heating means support **6** is heat-resistant. Typically, the heat-resistant heating means support **6** comprises, preferably consists essentially of, most preferably consists of, a material having a high RTI, typically above 200°C, preferably above 250°C, more preferably greater or equal to 270°C. Preferred heat-resistant heating means support **6** are those that comprise, preferably consist essentially of, most preferably consist of, a material selected from the group consisting of polyolefins (such as Polypropylene (PP) and Polyethylene (PE)), Polyamide (PA), Polyphenylene sulfide (PPS), Polytetrafluoroethylene (PTFE), Polyvinyltoluene (PVT), ceramics (such as porcelains and the like), and mixtures thereof, preferably, Polyphenylene sulfide (PPS), Polyamide (PA), and mixtures thereof. Alternatively other materials such as Propylene carbonate (PC) may be used, however in such instances, the PC is preferably coated with another material selected from the group consisting of of polyolefins (such as Polypropylene (PP) and Polyethylene (PE)), Polyamide (PA), Polyphenylene sulfide (PPS), Polytetrafluoroethylene (PTFE), Polyvinyltoluene (PVT), ceramics (such as porcelains and the like), and mixtures thereof.

The heating means support **6** may be made of a starting material that is not heat-resistant and subsequently treated to be heat-resistant, for example by coating the entire outer surface with a heat-resistant material.

It is preferred that the heat-resistant heating means support **6** is capable of maintaining the temperature of the housing **7** and/or the cartridge support **10** below the heat distortion temperature (HDT) (also known as the heat deflection temperature), preferably below 0.9HDT, more preferably below 0.8HDT, even more preferably below 0.7HDT, most preferably below 0.5HDT, preferably at the maximum operating temperature of the heating means. The maximum operating temperature typically being at least 90°C, preferably at least 95°C, more preferably at least 100°C, even more preferably at least 110°C, most preferably from greater than 120°C to 150°C. These operating conditions allow the housing to preserve its integrity even after an event of over-voltage. In a preferred embodiment the HDT of the housing and/or cartridge support is from 100°C to 200°C, measured according to AST D648 under a load of 0.45MPa.

In one embodiment, the heat-resistant heating means support **6** has a heat distortion temperature (HDT), of greater or equal to 270°C, measured according to AST D648 under a load of 1.8MPa.

The heating means support **6** may be arranged to displace from a first point up to a second point upon actuation of the actuating means **5,** which may be slidably connected to the housing **7,** wherein in the first point the distance between the heating means **5** and the wick **4** is from 0.50mm to 1.50mm, and wherein in the second point the distance between the heating means **5** and the wick **4** is from 5mm to 7mm. The actuating means **9** and/or the housing **7** may comprise friction means, preferably to provide physical or mechanical engagement at one or more, preferably a plurality of, set positions such that the actuating means **9,** and consequently the heating means support **6,** is held in place in said set position(s) once the actuating means **9** is released. The friction means may be in the form of one or more protrusions and/or recesses on a surface of the actuating means **9** and/or on a surface of the housing. The friction means may be arranged such to cause displacement between the actuating means **9** and the housing **7,** in a plane parallel or perpendicular to the wick height, as the actuating means **9** is actuated. The displacement being sufficient to cause a slight separation between the actuating means and the housing, such that a "click" sound is made once the actuating means slides over the friction means. This has the advantage that the user is alerted once the actuating means, and consequently the heating means support, has reached one of the set positions.

In a preferred embodiment, the heating means support **6** is arranged such that the distance, preferably the minimum distance, between the heating means **5** and the housing **7** is greater than 3mm, preferably from 3mm to 10mum, more preferably from 3mm to 7mm, most preferably from 3mm to 6mm. By "minimum distance" it is herein intended the distance from the heating means **5** to the closest point on the housing at any one position of the heating means support **6.**

In a preferred embodiment, the heating means **5** may have a number of different shapes, said shapes being selected from the group consisting of disc shaped, ring-like shaped, oval shaped, square shaped, rectangular shaped, pyramidal shaped, cube shaped, cuboid shaped, parallelepiped shaped, spherical shaped, and combinations thereof. Preferably, the heating means is parallelepiped in shape, preferably having a rectangular cross section, and may have substantially right angled edges. By "right angled edges" it is herein intended that the angle between respective faces formed by the outer surface of the heating means are substantially at 90°, preferably at 90°. Such ensures to minimize manufacturing complexity and costs whilst attaining the desired temperature outputs.

In a preferred embodiment, the heating means **5** is parallelepiped in shape having a rectangular cross section, the shorter length thereof being oriented parallel to the wick height. Preferably, said shorter length is the same length as the height of the wick **4** which remains directly exposed to the heat generated by the heating means. An advantage of this embodiment is that heat is effectively localized in the region of interest (i.e. only at the portion of the wick that remains directly exposed) thus limiting excess heat being provided in other regions of the device for a more efficient heating whilst limiting negative effects on other components of the device. Another advantage is that of device compaction.

The heating means **5** may comprise a plurality of faces, wherein one face of the heating means **5** is directly exposed to the wick **4** and wherein the remaining faces are directly in contact with the heating means support **6.** The face of the heating means **5** directly exposed to the wick **4** may have a shape selected from the group consisting of flat, concave, convex, and combinations thereof, preferably flat.

In a preferred embodiment, the heating means **5** is arranged such that the perimeter of the one face of the heating means **5** directly exposed to the wick **4** sits substantially flush to the heating means support **6** preferably such that only said one face is uncovered from said heating means support.

The heating means **5** preferably consists of an ceramic encased wire-wound resistor preferably made by winding resistive wire on fibre glass cord or ceramic rods and encapsulated in a ceramic body with temperature and fire retardant cement. These types of resistors are particularly cost efficient to manufacture while being able to dissipate large amount of heat.

In a preferred embodiment, the air treatment device **1** further comprises a cartridge support **10.** The cartridge support **10** may comprise a top and a bottom, wherein the top is juxtaposed to the heating means **5** and heating means support **6** and the bottom is juxtaposed the reservoir **3** of the replaceable cartridge **2,** when said air treatment device **1** is fully assembled. The cartridge support **10** may be enclosed within said housing **7.** Preferably the cartridge support **10** comprises a chimney **13** in fluid communication with the at least one opening of the housing **7** and is preferably located at said top and capable of receiving at least a portion of the wick **4** therein. The chimney **13** may be ring shaped or tubular in shape and sized such that a gap is left between said wick **4** and the inner surface of the chimney **13,** said gap preferably being sufficiently large to enable flow of vaporized air freshening substance through the chimney **13** and out of the air freshening device **1** via the at least one opening of the housing **7.** Preferably, the chimney **13** comprises at least one slit at a position proximal to the heating means such that the wick **4** remains directly exposed to the heat generated by the heating means **5.**

The cartridge support **10** is preferably a separate component directly connectable to the housing **7.** The cartridge support **10** is preferably interposed between the heating means **5** and at least a portion of the housing **7** and may be adapted to receive and retain the replaceable cartridge **2.** The cartridge support may comprise one or more polyesters, preferably a material selected from the group consisting of Polypropylene (PP), Polyethylene terephthalate (PET), Polyamide (PA), Polyoxymethylene (POM), and mixtures thereof, preferably Polypropylene (PP). In a preferred embodiment at least a portion of the cartridge support **10,** preferably the chimney **13** of the cartridge support **10,** is heat-resistant.

The air treatment device may further comprise a plug **14** for connection to a power source such as a power socket in a wall, car power jack and the like. Preferably, said plug **14** is connectable to a portion of the housing **7,** typically at a rear portion of the device, preferably slidably connected, such that the plug **14** is free to rotate about an axis of rotation perpendicular to the wick height. This ensures that the user may plug the device and orient it accordingly, to either fit into tight corners or ensure minimal risks of leakage by orienting the device upright, such that the replaceable cartridge sits below the heating means **5** and heating means support **6.** Alternatively, the air treatment device may comprise a battery compartment to receive one or more batteries, when the device is a battery powered device.

### REPLACEABLE CARTRIDGE

The air treatment device **1** according to the present invention comprises a replaceable cartridge **2.** The replaceable cartridge **2** permits the user to replace a used cartridge for a new one. The used cartridge may be replaced either when it is empty or if the user desires to quickly and easily change the fragrance vaporized by the air treatment device **1.**

Replaceable cartridges typically suitable for connection to the air treatment device **1** according to the present invention comprise a reservoir **3** containing an air freshening substance and a wick **4** in fluid, preferably liquid, communication with said reservoir **3.** The wick **4** being arranged such that the bottom end **4b** is within the reservoir **3** and is in contact with the liquid, and the top end **4a** projects out of the reservoir **3** such that it remains exposed to heat generated by the heating means **5** when the replaceable cartridge **2** is connected to a portion of the air treatment device **1.**

The replaceable cartridge **2** may comprise locking means (not shown) that interact with at least a portion of the housing **7** or the cartridge support **10** such that only cartridges comprising said locking means may be inserted and/or retained into the air treatment device, or such that only cartridges comprising said locking means enable the heating means 5 to apply direct heat to the wick **4** once connected to the air treatment device **1.**

The reservoir **3** of the replaceable cartridge may have any suitable shape and may be made of any suitable material for holding an air freshening substance without reacting with the components present in said air freshening substance. The reservoir may be made of glass or plastic and may be clear or opaque.

In a preferred embodiment, the replaceable cartridge comprises a single reservoir comprising one or more air freshening substances. When more than one air freshening substances are used, they may be contained in the single partitioned or partitionless reservoir.

Suitable wicks for air treatment devices according to the present invention are those of fibrous nature, synthetic or natural, capable of drawing the air freshening substance from the bottom end of the wick in the reservoir to the top end of the wick against gravity. Preferred wicks are sintered porous wicks comprising a material selected from the group consisting of polyolefins (such as PP and PE), Polyamide (PA), polyvinyl chloride (PVC), polylactic acid (PLA), polyethylene terephthalate (PET) and mixtures thereof, preferably Polypropylene (PP), Polyethylene (PE), polyethylene terephthalate (PET) and mixtures thereof.

Wicks can be described in terms of their average pore size. The wicks may have any suitable average pore size. US Patent Application 2002/0136886 A1, titled "Porous Wick for Liquid Vaporizers" provides a description of standard measurement of average pore size. Average pore size as referred to in the present invention, is measured by Mercury Intrusion Poresimetry as described in at least paragraphs 0028 and 0029 of US Patent Application 2002/0136886 A1. In certain embodiments, the average pore size of wicks useful in the present invention ranges from 50 microns to 500 microns, preferably from 50 microns to 200 microns, more preferably from 60 to 150, even more preferably from 70 microns to 100 microns. Similarly, wicks can have an average pore volume from 15 % to 85 %, preferably from 25 % to 50 %, as measured using Mercury Intrusion Poresimetry as described in at least paragraphs 0030 to 0035 of US Patent Application 2002/0136886 A1. Similarly, wicks can vary in length, depending on the desired use. In certain embodiments, wicks can have a length of from 30mm to 100mm, preferably from 40mm to 75mm, more preferably from 50mm to 70mm.

### THE AIR FRESHENING COMPOSITION

Air freshening compositions suitable for use in an air treatment device **1** according to the present invention are those which are preferably in liquid form, and are suitable to being subjected to heat.

Suitable liquid air freshening substances typically comprise one or more volatile components preferably selected from perfumes, malodor counteractants, solvents and mixtures thereof.

In certain embodiments, the air freshening substances described herein can maintain a more consistent character over time. The mixing action of the heavier, higher Odor Detection Threshhold ("ODT ") molecules (e.g., bottom notes such as musks, woody notes, etc.) with the newly released fresher more volatile lower ODT materials, will provide the user with a scent that is reminiscent of the one they initially experienced when the product was first applied. Without wishing to be bound by theory, it is believed that by increasing the level of base notes such as musks, woody notes etc., and carefully selecting such base notes having lower volatility, the character and noticeability of the fragrance is improved over a longer period of time. In a preferred embodiment the liquid air freshening substance comprises at least 10% pure raw materials (PRM) in the KI range of from 1500 to 1700 KI.

In one embodiment, the air freshening composition comprises one or more thickeners preferably selected from the group consisting of ethyl cellulose; low molecular weight polyethylene, such as ACumis B-6, B-12, B-18, C-5, C-12, and C-18, all manufactured by Honeywell; ethylene hompolymers such as A-C6, A-C6A, A-C7, A-C7A, A-C8, A-C8A, A-C9, A-C9A, A-C9F, A-C15, A-C16, A-C617, A-C715, and A-C1702, all from Allied Signal; micronized polyethylene wax, such as ACumist A-6, A-45, B-9, C-9, C-30, and D-9, all from Honeywell; styrenebutadiene-styrene triblock copolymers sold under the trade name Kraton by Shell Chemical Company; and mixtures thereof, preferably ethyl cellulose. Such has the advantage that likelihood of leakage or seepage is reduced.

### METHOD OF VAPORIZING AN AIR FRESHENING SUBSTANCE

The air treatment device according to the present invention may be used to vaporize an air freshening substance comprising in a method comprising the steps of:
i. optionally connecting the replaceable cartridge **2** to the air treatment device **1;**
ii. activating an air treatment device to start an activation period; and
iii. sequentially moving the actuating means **9** into a plurality of defined positions, wherein in each position the heating means support **6** is moved at a distance different to the previous position, for an effective time period in each said defined position, such that the evaporation rate of said air freshening substance is varied throughout said activation period.

In a preferred embodiment, step iii. comprises the steps of:
a. moving the actuating means **9** to a first position, such that the heating means support **6** is moved at a distance from the wick which is less than that when said air treatment device is activated, for a first period of time;
b. moving the actuating means **9** to a second position, such that the heating means support **6** is moved at a distance from the wick which is greater than that in said first position but different to that when said air treatment device is activated, for a second period of time that is sufficiently long to reduce the detection level of a human being to the level before habituation;
c. optionally moving the actuating means **9** to a third position, such that the heating means support **6** is moved at a distance from the wick which is less than that in said second position, for a third period of time wherein said third period of time is longer than said second period of time; and
d. optionally repeating steps a. to c.

The method described has the further advantage that noticeability of the vaporized air freshening substance in an open space remains detectable by a user thus limiting or even preventing habituation.

### RTI MEASUREMENT

The RTI as used herein is measured according to UL743B. According to UL743B (November 3, 2011), the relative thermal index (RTI) is the maximum temperature below which a material maintains its characteristics over a reasonable period. Unless specified otherwise, a 50-percent loss of the property value due to thermal degradation shall be considered as the property endpoint. Specific properties to be evaluated in a thermal-aging program are subdivided in electrical properties (e.g. dielectric strength), impact properties (e.g. tensile impact, Izod or Charpy impact) and structural properties (e.g. maximum tensile stress or flexural strength). Thermal indexes are assigned based on actual testing at thicknesses. Two sampling techniques are available at conducting a long-term thermal aging investigation: fixed temperature or fixed time frame methods; at least four oven temperatures have to be selected all with different time end points as specified in table 12.1 in page 13 of UL734B Aug 20, 2009. Specifically, the structural RTI can be determined by testing the candidate material samples according to standards ASTM D-638.

The dimensions and values disclosed herein are not to be understood as being strictly limited to the exact numerical values recited. Instead, unless otherwise specified, each such dimension is intended to mean both the recited value and a functionally equivalent range surrounding that value. For example, a dimension disclosed as "40 mm" is intended to mean "about 40 mm."

## Claims

1. An air treatment device (1) for the vaporization of an air freshening substance, said device comprising:
a replaceable cartridge (2) comprising a reservoir (3), for containing said air freshening substance, and a wick (4), in fluid communication with said reservoir (3), having a top end (4a) and a bottom end (4b) defining a wick height;
a heating means (5) for heating said wick (4) to a predetermined temperature to vaporize the air freshening substance drawn towards said top end (4a) by said wick (4);
a heating means support (6) for retaining said heating means (5); and
a housing (7) enclosing at least said heating means (5) and heating means support (6) therein, and
having at least one opening for allowing the vaporized air freshening substance to exit the air treatment device (1),
**characterized in that** at least a portion of the heating means support (6) is interposed between the housing (7) and the heating means (5) and wherein the heating means (5) is substantially interposed between said wick (4) and said heating means support (6), the heating means support (6) being arranged to displace in a direction perpendicular to said wick height, upon actuation of an actuating means (9) connectable to said heating means support (6), such that the evaporative rate of the air freshening substance is varied, and **in that** said heating means support (6) is heat-resistant.

2. An air treatment device (1) according to claim 1 wherein a substantial portion of the heating means support (6) is interposed between the housing (7) and the heating means (5), preferably the entire heating means support (6) is interposed between the housing (7) and the heating means (5).

3. An air treatment device (1) according to any of the preceding claims wherein one face of the heating means (5) is directly exposed to the wick (4) and wherein the remaining faces are directly in contact with the heating means support (6).

4. An air treatment device (1) according to any of the preceding claims wherein the air freshening substance consists of a liquid air freshening substance comprising one or more volatile components.

5. An air treatment device (1) according to any of the preceding claims wherein the heating means (5) consists of an encapsulated heater, preferably a ceramic and/or cement encapsulated heater, more preferably a wire-wound ceramic encapsulated positive temperature coefficient heating element.

6. An air treatment device (1) according to any of the preceding claims wherein the predetermined temperature is at least 85°C, preferably greater than 90°C, more preferably greater than 95°C.

7. An air treatment device (1) according to any of the preceding claims wherein the replaceable cartridge (2) comprises a single reservoir comprising one or more air freshening substances.

8. An air treatment device (1) according to any of the preceding claims wherein the heating means support (6) comprises a heat-resistant material capable of maintaining the temperature of the housing (7) below heat distortion temperature (HDT), preferably below 0.9HDT, more preferably below 0.8HDT, even more preferably below 0.7HDT, most preferably below 0.5HDT, of the housing (7).

9. An air treatment device (1) according to any of the preceding claims wherein the heating means support (6) is entirely made of a heat-resistant material selected from the group consisting of Polyphenylene sulfide (PPS), Polyamide (PA), and mixtures thereof.

10. An air treatment device (1) according to any of the preceding claims wherein the heating means support (6) is arranged to displace from a first point up to a second point upon actuation of the actuating means (5), wherein in the first point the distance between the heating means (5) and the wick (4) is from 0.5mm to 1.5mm, and wherein in the second point the distance between the heating means (5) and the wick (4) is from 5.0mm to 7.0mm.

11. An air treatment device (1) according to any of the preceding claims wherein the heating means support (6) is arranged such that the distance between the heating means (5) and the housing (7) is greater than 3mm, preferably from 3mm to 7mm, more preferably from 3mm to 6mm.

12. An air treatment device (1) according to any of the preceding claims wherein the heating means (5) is substantially parallelepiped in shape, preferably having substantially right-angled edges.

13. An air treatment device (1) according to any of the preceding claims further comprising a cartridge support (10) having a top and a bottom, wherein the top is juxtaposed to the heating means (5) and heating means support (6) and the bottom is juxtaposed the reservoir (3) of the replaceable cartridge (2), said cartridge support comprising a chimney (13), in fluid communication with the at least one opening of the housing (7), said chimney (13) located at said top and capable of receiving at least a portion of the wick (4) therein, preferably said cartridge support (10) being a separate component directly connectable to the housing (7), the cartridge support (10) being preferably interposed between the heating means (5) and a portion of the housing (7).

14. A method of vaporizing an air freshening substance comprising the steps of:
i. optionally connecting a replaceable cartridge to an air treatment device according to any of the preceding claims;
ii. activating an air treatment device (1) according to any of the preceding claims to start an activation period; and
iii. sequentially moving the actuating means (9) into a plurality of defined positions, wherein in each position the heating means support (6) is moved at a distance different to the previous position, for an effective time period in each said defined position, such that the evaporation rate of said air freshening substance is varied throughout said activation period.

15. A method according to claim 14 wherein step iii. comprises the steps of:
a. moving the actuating means (9) to a first position, such that the heating means support (6) is moved at a distance from the wick which is less than that when said air treatment device is activated, for a first period of time;
b. moving the actuating means (9) to a second position, such that the heating means support (6) is moved at a distance from the wick which is greater than that in said first position but different to that when said air treatment device is activated, for a second period of time that is sufficiently long to reduce the detection level of a human being to the level before habituation;
c. optionally moving the actuating means (9) to a third position, such that the heating means support (6) is moved at a distance from the wick which is less than that in said second position, for a third period of time wherein said third period of time is longer than said second period of time; and
d. optionally repeating steps a. to c.
